# EUROPEAN PATENT APPLICATION

(11) **EP 3 904 876 A1**
(43) Date of publication of application: **03.11.2021**
(21) Application number: 20382343.0
(22) Date of filing: 28.04.2020
(51) Int. Cl.: G01N 33/543, G01N 33/558

(54) **A FOLDABLE FLUIDIC DEVICE AND METHOD FOR BIOMARKER DETECTION IN BODY FLUIDS**

(71) Applicant: Universitat Politècnica De Catalunya, 08034 Barcelona (ES); Consejo Superior de Investigaciones Cientificas, 28006 Madrid (ES)
(72) Inventor: BASSEGODA PUIGDOMENECH, Arnau, 08011 Barcelona (ES); HOYO PÉREZ, Javier, 08014 Barcelona (ES); TZANOV, Tzanko, 08223 Terrassa (ES); FERNÁNDEZ SÁNCHEZ, César, 08206 Sabadel (ES); GUTIÉRREZ CAPITÁN, Manuel, 08191 Rubí (ES); BALDI COLL, Antonio, 08172 Sant Cugat del Vallès (ES)
(74) Representative: Juncosa Miró, Jaime

(57) **Abstract**

A foldable fluidic device and a method for biomarker detection in body fluids are provided. The device comprises a substrate comprising at least two independent foldable sections including a hydrophobic layer that establishes a three-dimensional fluidic pattern defining a plurality of areas. The areas comprise a sample application area to receive a fluid sample, a first immobilization area to immobilize a first sensing reagent against a biomarker, a second immobilization area to immobilize a second sensing reagent, an absorbent area, and channels allowing the fluid sample to flow from the application area towards the absorbent area. In response to an interaction of the second sensing reagent with a product obtained as a result of the interaction of the first sensing reagent with the fluid sample, the second immobilization area can change its color when a concentration of the biomarker, equal or above a threshold, is detected in the fluid sample.

## Description

### Technical Field

The present invention is directed, in general, to the field of fluidic devices. In particular, the invention relates to a foldable fluidic device and to a method for biomarker detection in body fluids.

### Background of the Invention

Conventional procedures, such as microbiological test, are time consuming or show limited reliability due to the subjective judgement of the classical clinical signs - redness, heat, swelling, and pain. The fast diagnosis based on the objective evaluation of biomarkers permits an accurate treatment which results in a faster recovery from the unhealthy situation. As in other fields, the fast evaluation of the situations renders a fast response and diminishes the damage. Several approaches have been developed for evaluation of biomarkers from biological samples, however most of them require the use of complex instruments by expert technicians and are time or money consuming. Hence, the medical practitioners are interested in the development of simple and cheap point of care testing (PoCT) devices for fast and objective diagnosis. In the last decade, chemically patterned paper has shown great potential for the development of cheap versatile fluidic devices that can be used as PoCT devices. Paper is cheap and biodegradable, its porous cellulose structure wicks fluids without the requirement of a pumping mechanism and is usually white, being the best platform for colorimetric detection.

Apart from that, chronic wounds represent a challenge to wound care professionals and consume a great deal of healthcare resources around the globe. The severity and cost of wound infections increase dramatically the longer they remain untreated. The resulting pain, impairment and social isolation lead to reduced quality of life and, in the worst case, hospitalization, and eventually sepsis and death. Heavy bacterial colonization is one of the main reasons for non-healing of chronic wounds, therefore an early detection of an incipient wound infection by the attending physician is crucial for the choice of appropriate treatment to reduce the severity of the disease and avoid the chronicity. Standard procedures for wound infection detection are time consuming (microbiological tests) or show limited reliability due to the subjective judgement of the classical clinical signs. There is interest in the design of novel PoCT devices based on the objective evaluation of infection biomarkers to achieve fast and reliable diagnostic on the wound incipient infection. For example, Myeloperoxidase (MPO) is an enzyme released by activated neutrophils. MPO catalyzes the oxidation of chloride ions, using hydrogen peroxide as co-substrate, to produce hypochlorous acid (HCIO), a powerful bactericidal oxidant. MPO shows about ten times higher activities in infected wounds compared to non-infected. Therefore, the detection of this enzyme by PoCT has been postulated as a useful biomarker for wound diagnostics.

Scientific document *"*New myeloperoxidase detection system based on enzyme-catalyzed oxidative synthesis of a dye for paper-based diagnostic devices*",* of the same inventors of present invention, reports a system for MPO detection based on enzyme-catalyzed oxidative synthesis of a dye. The published research work shows the formation of a purple dye due to the MPO-mediated oxidation of dye precursors using hydrogen peroxide as co-substrate. The dye precursors can be incorporated on paper substrate where the color formation can be clearly observed. The obtained dye was fully characterized determining the molecular weight and empirical formula. Present invention, on the contrary, relates to a fluidic device comprising a single foldable substrate for detection of different biomarkers which is designed to incorporate two detection events, thus achieving higher detection specificity. As a particular example of the device use, the detection of MPO is presented. To achieve such detection, the proposed device is functionalized with the specific antibody to selectively immunocapture MPO (first detection event) and the aforementioned published colorimetric strategy is used to reveal the presence of the immunocaptured MPO (second detection event). This detection example with the proposed device illustrates its functioning principles which allow the detection of other biomarkers through the incorporation of specific detection events.

Patent application US 20193292-A1 provides a paper-based detecting platform having a foldable and slidably multi-layer vertical flow design. The paper-based vertical flow test device comprises: a detection portion having a test zone and a control zone for receiving a specimen fluid containing a target protein; a conjugation portion having an enzyme-labelled protein, the conjugation portion being movably disposed above the detection portion to allow the conjugation portion to be pulled away from the detection portion or to cover the detection portion; and an absorbent portion having a multi-layer structure and being disposed below the detection portion, wherein the absorbent portion has a first absorbing zone and a second absorbing zone corresponding to the test zone and the control zone, respectively.

New fluidic devices and methods for biomarker detection in body fluids having simpler, single-use and low-cost designs/configurations are therefore needed.

### Description of the Invention

To that end, the present invention proposes, according to one aspect, a foldable fluidic device for biomarker detection in body fluids. The proposed device comprises a substrate including at least two independent foldable sections; said substrate including a hydrophobic layer that forms a three-dimensional fluidic pattern, for example a microfluidic pattern, defining a plurality of areas; said plurality of areas comprising: a sample application area to receive a body fluid sample, a first immobilization area to immobilize at least one first sensing reagent against a given biomarker, a second immobilization area to immobilize at least one second sensing reagent, an absorbent area, and channels allowing the body fluid sample to flow from the sample application area towards the absorbent area through the first immobilization area.

In response to a contact of the second sensing reagent with a product obtained as a result of the interaction of the at least one first sensing reagent with the body fluid sample, caused by the folding of said the at least two independent foldable sections, the second immobilization area is adapted to change its color if a concentration of the given biomarker detected in the body fluid sample is equal to or greater than a threshold.

In an embodiment, the substrate comprises a paper substrate, for example made of a cellulose or nitrocellulose fiber, among others. Alternatively, in other embodiments, the substrate can be of different materials, for example of a polymer such as poly(L-lactic acid) or poly(vinylidene fluoride).

In an embodiment, the substrate comprises two independent foldable sections, a first foldable section and a second foldable section. In this case, the sample application area, the first immobilization area and the absorbent area are directly connected by the channels in the first foldable section, whereas the second immobilization area is located in the second foldable section.

In another embodiment, the substrate comprises three independent foldable sections, a first foldable section, a second foldable section and a third foldable section. In this case, the channels are located in the first foldable section; the sample application area, the first immobilization area and the absorbent area are located in the second foldable section; and the second immobilization area is located in the third foldable section. In a folded position the channels connect the sample application, the first immobilization and the absorbent areas.

In some embodiments, the absorbent area can include a reagent to produce a color signal upon wetting.

In an embodiment, the first sensing reagent comprises an antibody against myeloperoxidase, and the second sensing reagent comprises enzyme glucose oxidase, m-phenylenediemine, 2,2'-azino-bis(3-ethylbenzothiazoline-6-sulfonic acid) diammonium salt and branched polyethyleneimine. In this particular case, the second immobilization area is adapted to change its color to purple upon said myeloperoxidase detection.

In an embodiment, the sample application area, the first immobilization area, the absorbent area and the channels further comprise glucose and a fluidity improving agent including a given concentration of bovine serum albumin and tween.

In an embodiment, the proposed device also includes a casing, for example made of a polymer or metallic material, to hold the substrate. In some embodiments, the casing can be made of a flexible material hence allowing the folding of the different foldable sections in order to allow the biomarker detection.

Embodiments of the present invention also provide, according to another aspect, a method for biomarker detection in body fluids using a foldable fluidic device. The method comprises providing a substrate comprising at least two independent foldable sections; depositing a hydrophobic material on a surface of the substrate, the hydrophobic material penetrating across a whole thickness of the substrate establishing a three-dimensional fluidic pattern defining a plurality of areas, said plurality of areas comprising a sample application area, a first immobilization area, a second immobilization area, an absorbent area and channels; functionalizing the first immobilization area with at least one first sensing reagent against a given biomarker and functionalizing the second immobilization area with at least one second sensing reagent; applying a body fluid sample to said sample application area, the body fluid sample flowing from the sample application area towards the absorbent area through the first immobilization area thanks to the channels, wherein a product is generated in the first immobilization area as a result of the reaction of the body fluid sample with the at least one first sensing reagent; putting into contact the second immobilization area with the first immobilization area, during a given time, by folding said at least two independent foldable sections; and obtaining a biomarker detection result as a result of a change of color of the second immobilization area, said change of color occurring when a concentration of the given biomarker is detected in the body fluid sample equal to or greater than a threshold.

In an embodiment, in particular for the detection of the biomarker myeloperoxidase, the first sensing reagent comprises an antibody against myeloperoxidase; the second sensing reagent comprises enzyme glucose oxidase, m-phenylenediemine, 2,2'-azino-bis(3-ethylbenzothiazoline-6-sulfonic acid) diammonium salt and branched polyethyleneimine; and
the color change of the second immobilization area is purple.

In an embodiment, the body fluid sample is applied to said sample application area after a first foldable section of said at least two independent foldable sections is folded over or under a second foldable section of said at least two independent foldable sections, wherein the first foldable section comprises the channels and the second foldable section comprises the sample application area, the first immobilization area and the absorbent area.

In an embodiment, the second immobilization area and the first immobilization area are put into contact after a color indication signal appears in the absorbent area.

In yet another embodiment, the method comprises adding glucose and a fluidity improving agent to the sample application area, the first immobilization area, the absorbent area and the channels, the fluidity improving agent particularly comprising a given concentration of bovine serum albumin and tween.

Therefore, present invention allows the implementation of biomarker detection strategies based on two detection events for increased specificity thus going beyond the available devices that offer just a single detection step such as an immunodetection. In addition, the versatile detection platform presented here provides a single use and cost efficient solution that can be used without technical knowledge.

### Brief Description of the Drawings

The previous and other advantages and features will be more fully understood from the following detailed description of embodiments, with reference to the attached figures, which must be considered in an illustrative and non-limiting manner, in which:
Fig. 1 illustrates a foldable fluidic device for biomarker detection in body fluids, according to an embodiment of the present invention.
Fig. 2 illustrates another foldable fluidic device for biomarker detection in body fluids, according to an embodiment of the present invention.
Fig. 3 illustrates the folding sequence for the foldable fluidic device, according to an embodiment of the present invention.

### Detailed Description of Preferred Embodiments

Present invention provides a foldable fluidic device and a method for biomarker detection in body fluids. The proposed device is a single-use cheap foldable fluidic device that allows the incorporation of sensing molecules, e.g. enzyme substrates and antibodies, in specific spots/areas delimited within the device where they will react with the target biomarker when the fluid or liquid sample flows through the device.

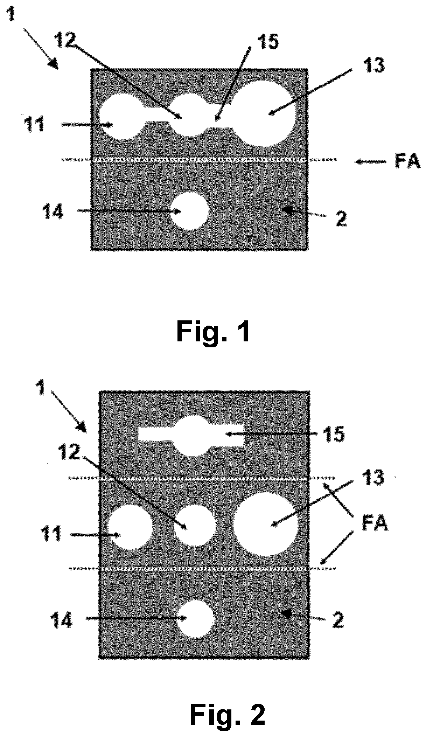

Fig. 1 shows a first embodiment of the proposed device. According to this first embodiment, the device is formed by a single substrate 1 that is foldable via one foldable axis FA. The substrate 1 can be a paper substrate or a polymeric substrate and has the size of a credit card, or even smaller. In a particular embodiment the substrate is paper-based.

The substrate 1 comprises a hydrophobic layer 2. The hydrophobic layer 2 can be formed after having deposited a hydrophobic material on the substrate 1. Incubation at controlled temperature allowed penetration of the hydrophobic material through the whole cellulose material thickness, thus achieving efficient liquid flow control by the hydrophobic pattern. A three-dimensional fluidic pattern defines the areas through which the body fluid sample will flow and eventually react with immobilized sensing reagents.

As seen in Fig. 1 the areas include: a sample application area 11, a first immobilization area 12, a second immobilization area 14, an absorbent area 13 and channels 15. In this embodiment, the sample application area 11, the first immobilization area 12 and the absorbent area 13 are directly connected by the channels 15.

With reference now to Fig. 2, therein it is illustrated another embodiment of the proposed device. Different to the embodiment shown in Fig. 1, in this case the substrate 1 is foldable via two foldable axes FA. The channels 15 are placed in a different zone than the sample application 11, the first immobilization 12 and the absorbent 13 areas and upon folding connect said areas.

In either of the embodiments of Fig. 1 or 2, the second immobilization area 14 is placed in an independent foldable section of the substrate 1, without direct connection with the other areas.

After defining the functional fluidic pattern, functionalization of the substrate 1 is performed. The first 12 and second 14 immobilization areas are functionalized with first and second sensing reagents, respectively.

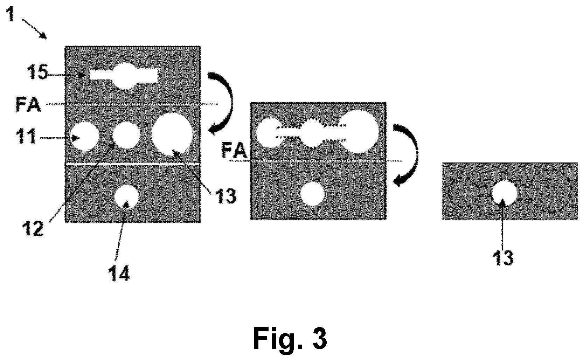

The three-dimensional fluidic pattern design allows the separation in space and time of two sequential events for the detection of the desired biomarker. This separation allows implementing complementary biomarker detection reactions to increase the detection specificity while avoiding possible interferences. The first event involves the sample application area 11, the first immobilization area 12, the absorbent area 13 and the channels 15. The body fluid sample is applied to the sample application area 11, initiating the flow, by capillarity, towards the absorbent area 13 through the first immobilization area 11 thanks to the channels 15. When the flowing sample encounters the immobilized sensory reagents in the first immobilization area 11 the first event such as an immunocapture by immobilized antibodies is triggered. The substrate 1 can be folded (see Fig. 3), by a user, to put in contact the first 12 and second 14 immobilization areas, which should remain in contact for a determined incubation time. Once these two areas 12, 14 are in contact, the second immobilization area 14 is wetted by the first immobilization area 12 and the product of the first event, such as a captured biomarker, is exposed to the sensory molecules of the second immobilization area 14 triggering a second event. This step aims at producing a clear signal to visually infer the presence of the detected biomarker for example a color development reaction catalyzed by an immunocaptured enzyme during the first event.

In some embodiments, the absorbent area 13 can be also functionalized with a reagent, for example a colorant, which changes the color upon wetting. Hence, in this case, the folding of the substrate 1, by a user, to put in contact the first 12 and second 14 immobilization areas can be triggered by a change of color of the absorbent area 13.

Furthermore, in some embodiments, at least the sample application area 11, the first immobilization area 12 and the absorbent area 13 can be functionalized to improve the sample flow and avoid unspecific and undesired interaction between the components of body fluid sample, such as the biomarker of interest and the cellulose support.

Following, an example of use of the proposed device for detection of biomarker myeloperoxidase (MPO) is described.

Prior to device use, the first 12 and second 14 immobilization areas are functionalized with different sensing reagents followed by drying process. Antibodies against MPO are immobilized in the first immobilization area 12. In the second immobilization area 14 enzyme glucose oxidase (GOX), m-phenylenediemine (mPD), 2,2'-azino-bis(3-ethylbenzothiazoline-6-sulfonic acid) diammonium salt (ABTS) and branched polyethyleneimine are deposited. Also, the sample application area 11, the first immobilization area 12, the absorbent area 13 and the channels 15 are functionalized with glucose and a fluidity improving agent comprising bovine serum albumin and tween (e.g. tween® 20). The liquid sample, such as wound fluid from a chronic wound, is applied at the application area 11, activating the flow caused by the channels 15 towards the absorbent area 13 via first immobilization area 12. The flowing sample encounters the immobilized antibodies which capture the MPO. When the absorbent area 13 is filled with flowing sample (color change of the absorbent area 13) the device can be folded to contact the second immobilization area 14 with the first immobilization area, maintaining this contact for a given time (for example 5 minutes). The second immobilization area 14 is wetted by the first immobilization area 12 which trigger the following reactions. The glucose in the first immobilization area 12 is oxidized by GOX of the second immobilization area 14 producing hydrogen peroxide. Simultaneously the immunocaptured MPO in the first immobilization area 12 uses the generated hydrogen peroxide to oxidize the sensing reagents ABTS and mPD of the second immobilization area 14 and produce a purple color clearly distinctive by naked eye. The concentration of the sensing reagents immobilized on the second immobilization area 14 are optimized to obtain, after a given time interval, for example after 5 minutes of incubation, a distinctive purple color development only at MPO concentrations identified in infected chronic wounds while no color change when the liquid sample contains MPO concentration present in non-infected wounds.

In some embodiments, the proposed device also includes a casing to hold the foldable substrate 1. The casing can be made of a rigid or flexible polymeric material. For example, the casing in some embodiments can be made of a flexible polymeric material allowing the folding of the substrate 1 as previously described.

In other embodiments, the casing is rigid and it is only used to protect the substrate 1, for instance, during transport thereof. In this latter case, other material different from a polymer could be used, for example a metal, among others.

The scope of the present invention is defined in the following set of claims.

## Claims

1. A foldable fluidic device for biomarker detection in body fluids, comprising:
a substrate (1) comprising at least two independent foldable sections;
said substrate (1) includes a hydrophobic layer (2) that establishes a three-dimensional fluidic pattern defining a plurality of areas;
said plurality of areas comprises: a sample application area (11) adapted to receive a body fluid sample, a first immobilization area (12) adapted to immobilize at least one first sensing reagent against a given biomarker, a second immobilization area (14) adapted to immobilize at least one second sensing reagent, an absorbent area (13), and channels (15) allowing the body fluid sample to flow from the sample application area (11) towards the absorbent area (13) through the first immobilization area (12); and
wherein in response to a contact of the second sensing reagent with a product obtained as a result of the interaction of the at least one first sensing reagent with the body fluid sample, caused by the folding of said at least two independent foldable sections, the second immobilization area (14) is adapted to change its color when a concentration of the given biomarker detected in the body fluid sample is equal or above a threshold.

2. The device of claim 1, wherein the substrate (1) comprises two independent foldable sections, a first foldable section and a second foldable section, wherein the sample application area (11), the first immobilization area (12) and the absorbent area (13) are directly connected by the channels (15) in said first foldable section, and wherein the second immobilization area (14) is located in said second foldable section.

3. The device of claim 1, wherein the substrate (1) comprises three independent foldable sections, a first foldable section, a second foldable section and a third foldable section, wherein the channels (15) are located in said first foldable section; the sample application area (11), the first immobilization area (12) and the absorbent area (13) are located in said second foldable section; and the second immobilization area (14) is located in said third foldable section, and wherein in a folded position the channels (15) connect the sample application (11), the first immobilization (12) and the absorbent (13) areas.

4. The device of any of previous claims, wherein the absorbent area (13) further includes a reagent to produce a color signal upon wetting.

5. The device of any of previous claims, wherein the first sensing reagent comprises an antibody against myeloperoxidase, and wherein the second sensing reagent comprises enzyme glucose oxidase, m-phenylenediemine, 2,2'-azino-bis(3-ethylbenzothiazoline-6-sulfonic acid) diammonium salt and branched polyethyleneimine.

6. The device of claim 5, wherein the second immobilization area (14) is adapted to change its color to purple upon said myeloperoxidase detection.

7. The device of any of previous claims, wherein the sample application area (11), the first immobilization area (12), the absorbent area (13) and the channels (15) further comprise glucose and a fluidity improving agent including a given concentration of bovine serum albumin and tween.

8. The device of any of previous claims, wherein the substrate (1) is made of a material comprising paper or a polymer.

9. The device of any of previous claims, further comprising a casing to hold the substrate (1).

10. A method for biomarker detection in body fluids using a foldable fluidic device, the method comprises:
providing a substrate (1) comprising at least two independent foldable sections;
depositing a hydrophobic material on a surface of the substrate (1), the hydrophobic material penetrating across a whole thickness of the substrate (1) forming a hydrophobic layer (2) that establishes a three-dimensional fluidic pattern defining a plurality of areas, said plurality of areas comprising a sample application area (11), a first immobilization area (12), a second immobilization area (14), an absorbent area (13) and channels (15);
functionalizing the first immobilization area (12) with at least one first sensing reagent against a given biomarker and functionalizing the second immobilization area (14) with at least one second sensing reagent;
applying a body fluid sample to said sample application area (11), the body fluid sample flowing from the sample application area (11) towards the absorbent area (13) through the first immobilization area (12) thanks to the channels (15), wherein a product is generated in the first immobilization area (12) as a result of the reaction of the body fluid sample with the at least one first sensing reagent;
putting into contact the second immobilization area (14) with the first immobilization area (12), during a given time, by folding said at least two independent foldable sections; and
obtaining a biomarker detection result as a result of a change of color of the second immobilization area (14), said change of color occurring when a concentration of the given biomarker is detected in the body fluid sample equal to or greater than a threshold.

11. The method of claim 10, wherein:
the first sensing reagent comprises an antibody against myeloperoxidase;
the second sensing reagent comprises enzyme glucose oxidase, m-phenylenediemine, 2,2'-azino-bis(3-ethylbenzothiazoline-6-sulfonic acid) diammonium salt and branched polyethyleneimine; and
the color is purple.

12. The method of claim 10 or 11, wherein the body fluid sample is applied to said sample application area (11) after a first foldable section of said at least two independent foldable sections is folded over or under a second foldable section of said at least two independent foldable sections, wherein the first foldable section comprises the channels (15) and the second foldable section comprises the sample application area (11), the first immobilization area (12) and the absorbent area (13).

13. The method of any of previous claims 10 to 12, wherein the second immobilization area (14) and the first immobilization area (12) are put into contact after a color indication signal appears in the absorbent area (13).

14. The method of any of previous claims 10 to 13, further comprising adding glucose and a fluidity improving agent to the sample application area (11), the first immobilization area (12), the absorbent area (13) and the channels (15), the fluidity improving agent comprising a given concentration of bovine serum albumin and tween.
